Europäisches Patentamt

**European Patent Office**

**Office européen des brevets**

(11) Publication number: **0 055 313**
**A1**

(12) # EUROPEAN PATENT APPLICATION

(21) Application number: **80304761.2**

(22) Date of filing: **30.12.80**

(51) Int. Cl.³: **A 61 K 9/02**

(43) Date of publication of application:
**07.07.82 Bulletin 82/27**

(84) Designated Contracting States:
**BE CH DE FR IT LI NL SE**

(71) Applicant: **Ed. Geistlich Söhne A.G. für Chemische Industrie**

**CH-6110 Wolhusen Lucerne(CH)**

(72) Inventor: **Rhodes, John**
**25 Nant Fawr Road**
**Cyncoed Cardiff(GB)**

(72) Inventor: **Evans, Brian Kenneth**
**9 Merevale The Common**
**Dinas Powis South Glamorgan(GB)**

(72) Inventor: **Heatley, Richard Val**
**1 Coed y Llyn**
**Cyncoed Cardiff(GB)**

(74) Representative: **Holmes, Michael John**
**Frank B. Dehn & Co. Imperial House 15-19 Kingsway**
**London WC2B 6UZ(GB)**

(54) Guaiacol and derivatives; compositions thereof and use in a method of treatment.

(57) The invention relates to a pharmaceutical composition for the treatment of heartburn by oral administration or the treatment of constipation of the large bowel by rectal administration which composition comprises guaiacol or a pharmaceutically acceptable derivative thereof and (a) at least one alkaline compound suitable for neutralising gastric acids and/or a mucilage compatible with the alimentary tract and/or a silicone antifoam or (b) a suppository mass. Guaiacol and its derivatives, formulated into pharmaceutical compositions have surprisingly been found to be useful in the treatment of heartburn or constipation.

- 1 -

Guaiacol and derivatives ; compositions thereof and use in a method of treatment.

The present invention relates to pharmaceutical compositions for the treatment of heartburn (irritation of the oesophagus) and of the large bowel.

Heartburn is a common clinical condition in patients with indigestion, due to the reflux of gastric contents into the lower oesophagus. This is sometimes associated with a low tone in the sphincter at the gastro-oesophageal junction (the lower oesophageal sphincter) or no sphincter at all. The current treatment for this condition consists of administering an alkali medicine, or an alkali in an alginate, which tends to adhere to the oesophageal wall and float on top of gastric fluid. It is claimed that the alkali material refluxes into the oesophagus and reduces irritation of the oesophagus by gastric acids.

Guaiacol, otherwise o-methoxy phenol, is a naturally occurring oil which at one time was used in expectorant preparations but which has fallen into disuse and no longer is listed in the British Pharmacopoeia. Certain guaiacol derivatives, such as guaiaphenesin, otherwise 3-(o-methoxyphenoxy)-propane-1,2-diol, have been proposed for use in expectorant preparations but the effect of these or guaiacol on the oesophageal sphincter or the large bowel has not been described nor indeed has the use of these compounds been suggested as a treatment for heartburn or the bowel.

We now have surprisingly found that guaiacol and derivatives thereof have the hitherto unsuspected property of increasing the tone of the lower oesophageal sphincter and that these compounds effect an increase in the size of propulsive contractions in the oesophagus. Furthermore it is thought that the compounds probably increase antral contractions and may increase the rate of gastric emptying. Moreover we have also found guaiacol and derivatives thereof to exhibit a useful effect on the activity of the large bowel, where it markedly increases motility and may be used to combat or alleviate the effects of constipation.

According to one aspect of the invention we provide a pharmaceutical composition for the treatment of heartburn by oral administration or the treatment of constipation of the large bowel by rectal administration which composition comprises guaiacol or a pharmaceutically acceptable derivative thereof and (a) at least one alkaline compound suitable for neutralising gastric acids and/or a mucilage compatible with the alimentary tract and/or a silicone antifoam or (b) a suppository mass.

The alkaline compound may be any such compound suitable for oral administration and effective for neutralising gastric acids. Among the alkaline compounds known to be useful for this purpose are aluminium hydroxide, magnesium hydroxide, magnesium trisilicate and mixtures of two or more of these.

In place of, or in addition to, guaiacol in the compositions of the invention, may be used pharmaceutically acceptable derivatives thereof such as guaiacol carbonate, guaiacol benzoate, guaiacol phosphate and, most suitably, the glyceryl ether guaiaphenesin.

In oral administration of the compositions of the present invention the guaiacol or derivative thereof may act to reduce the risk of reflux of gastric contents into the lower oesophagus, to increase the clearance rate of fluid from the oesophagus and to accelerate gastic emptying.

The mucilage component of compositions of the present invention may act to improve the adherence of the guaiacol or derivative to the lining of the alimentary tract. Gums suitable for forming the mucilage component of the compositions of the invention include, for example, acacia (gum arabic), tragacanth, methylcellulose, sodium carboxymethylcellulose, karoya (sterculia gum), alginic acid and alginates (such as sodium alginate). These gums, which at lower concentrations may act as suspending agents, will at higher concentration in an aqueous medium produce a mucilage. Any mucilage or suspending agent used should, of course, be palatable to ensure the composition is acceptable to the patient on oral administration.

As the above-mentioned alkaline compounds are frequently substantially water-insoluble powders, at least in the case of the orally administered compositions of the invention it may prove convenient to utilize a suspending agent to hold the powder in suspension and to maintain the guaiacol or its derivative dispersed in an aqueous medium.

Suitable suspending agents include the various gums mentioned above, used at relatively low concentrations, so as to assist suspension without rendering the composition unacceptably viscous. A suitable suspending agent is a food grade polysaccharide gum such as a xanthan gum. A particularly suitable commercial xanthan gum is that sold under the trade mark KELTROL which is stable over a wide range of pH values, which has a good mouth feel and masks any grittiness of the alkaline compound.

The compositions of the invention may also include additives for various purposes e.g. for the orally administered compositions preservatives (such as methyl and propyl p-hydroxybenzoate), sialagogues (such as citric acid), carriers (such as sorbitol and lactose), sweeteners (such as syrups) and flavours, and for the rectally administered compositions preservatives and carriers. As the suppository mass for rectally administrable compositions of the invention in the form of suppositories may conveniently be used cocoa butter or propylene glycol.

Those compositions of the invention which on administration are in the form of a solution, dispersion or suspension in an aqueous medium may be prepared by the addition of water to a mixture comprising the non-aqueous ingredients of the composition of the invention. Such mixtures of the non-aqueous ingredients of the compositions of the invention are also deemed to fall within the scope of this invention.

According to a further aspect of the present invention we provide a method of treatment of heartburn or of constipation of the large bowel by the oral or rectal administration respectively of an effective quantity of guaiacol or a pharmaceutically acceptable derivative thereof, preferably in the form of a composition according to the invention.

According to a further aspect of the present invention we provide guaiacol and its pharmaceutically acceptable derivatives, especially guaiaphenesin, for use in the treatment of heartburn or of constipation of the large bowel.

The compositions of the invention may be in dosage unit form, such as tablets, sachets or suppositories. Suitable doses of the principal components to be contained in each dosage unit are given below. Where the compositions are provided in bulk form, as in syrups, emulsions, enemas etc., the doses given below relate to a 20 ml volume; where it is recommended that larger or smaller volumes of such liquids would be more appropriate, the concentrations of the components concerned may be calculated to give the stated doses in the recommended volume of liquid.

The guaiacol or guaiacol derivative component of the compositions of the invention should advantageously be present at concentrations of from about 10 to about 1000 mg per dose ; in the case of guaiacol itself preferably from 15 to 100, most preferably about 60 mg per dose ; in the case of guaiaphenesin preferably from 120 to 500, most preferably about 300 mg per dose.

Where a silicone antifoam is present, this may be dimethicone, which may contain finely divided silica. Each dose of the composition conveniently contains 100 to 500 mg of antifoam.

Where the alkaline compounds are present in the compositions of the invention, these are present advantageously at concentrations of from about 0.2 to 1.6, preferably about 1 g per dose. The alkaline compounds are preferably in the form of a mixture of magnesium and aluminium compounds (suitably magnesium and aluminium hydroxides) in a weight ratio of from 5:7 to 1:1 of magnesium compound to aluminium compound.

In those compositions of the invention where a mucilage is present the gum from which the mucilage is formed, advantageously selected from alginic acid, alginates, tragacanth, gum arabic, methyl cellulose and karaya gum (or sterculia gum), should of course be in concentrations sufficient to impart the desired mucilagenous nature of the compositions. In the case where roughly equal parts by weight of alginic acid and sodium alginate are used, the alginic acid / alginate concentration will conveniently be about 500 to 1000, preferably about 800 mg per dose.

The compositions of the invention for oral administration may be prepared and packaged in forms such as powders, tablets, solutions, suspensions, emulsions and syrups. However, on administration it is preferred that the compositions be in aqueous liquid form a dose of which being for example about 20 ml.

The compositions of the invention for rectal administration may again be prepared and packaged in many forms but it is preferred that these be administered either

- 7 -

as suppositories or in liquid form, eg. in an enema.

The following Examples are provided to illustrate the present invention without serving to limit the scope of protection sought therefor:

Example 1

Aqueous preparation comprising the following ingredients:

| | |
|---|---|
| Guaiacol | 0.1 g |
| Alkaline compound | 8 g |
| (Aluminum hydroxide and magnesium hydroxide powder in equal weights) | |
| KELTROL (xanthan gum suspending agent) | 0.8 g |
| Preservative | 0.15g |
| (methyl and propyl p-hydroxybenzoates in 10:1 ratio by weight) | |
| Sialagogue | 1.0 g |
| (citric acid B.P.) | |
| Sweetener | 10 ml |
| (syrup B.P.) | |
| Water                          ad | 100 ml |

The composition of this Example would be administered orally in doses of about 20 ml.

Example 2

Aqueous preparation comprising the following ingredients:

| | |
|---|---|
| Guaiaphenesin | 1 g |
| Alkaline compound | 4 g |
| (Aluminium and magnesium hydroxide in powder form and in 7:5 weight ratio) | |
| KELTROL | 0.8 g |

(Xanthan gum suspending agent)

| | | |
|---|---|---|
| Preservative | | 0.15 g |
| Citric acid B.P. | | 1.0 g |
| Syrup B.P. | | 10 ml |
| Water | ad | 100 ml |

The composition of this Example would be administered orally in doses of about 20 ml.

Example 3.

Preparation, for admixing with water before oral administration, comprising the following ingredients:

| | |
|---|---|
| Guaiaphenesin | 0.25 g |
| Aluminium hydroxide | 0.2 g |
| Magnesium hydroxide | 0.2 g |
| sodium alginate | 0.4 g |
| alginic acid | 0.4 g |
| sweetener | 2 g |

The powdered composition may be made up into sachets or tablets each of which contains a single dose of the above concentrations.

Example 4.

Suppositories, each comprising the following ingredients:

| | | |
|---|---|---|
| Guaiaphenesin | | 0.25 g |
| Suppository mass | ad | 4.0 g |

Example 5.

    Tablets comprising the following ingredients per tablet:

| | |
|---|---|
| Guaiaphenesin | 0.25 g |
| Dimethicone | 0.25 g |
| Sorbitol | 0.40 g |

Example 6.

    Tablets comprising the following ingredients per tablet:

| | |
|---|---|
| Guaiacol | 0.10 g |
| Dimethicone | 0.30 g |
| Aluminium Hydroxide | 0.25 g |
| Sorbitol | 0.25 g |

CLAIMS

1.    A pharmaceutical composition for the treatment of heartburn by oral administration or the treatment of constipation of the large bowel by rectal administration which composition comprises guaiacol or a pharmaceutically acceptable derivative thereof and (a) at least one alkaline compound suitable for neutralising gastric acids and/or a mucilage compatible with the alimentary tract and/or a silicone antifoam or (b) a suppository mass.

2.    A composition as claimed in claim 1 wherein the said guaiacol derivative is guaiaphenesin.

3.    A composition as claimed in either of claims 1 and 2 wherein the said alkaline compounds are a 5:7 to 1:1 ratio by weight mixture of alkaline magnesium and aluminium compounds.

4.    A composition as claimed in any one of claims 1 to 3 wherein the said mucilage is an aqueous solution of at least one of the following : alginic acid, alginates, gum tragacanth, gum arabic, methyl cellulose, karaya or sterculia gum, and sodium carboxymethylcellulose.

5.    A composition as claimed in any one of claims 1 to 4 in dosage unit form comprising from 10 to 1000 mg per dose of guaiacol or a pharmaceutically acceptable derivative thereof.

6.    A composition as claimed in any one of claims 1 to 5 in dosage unit form comprising from 0.2 to 1.6 g per dose of the said alkaline compound.

7.    A composition as claimed in any one of claims 1 to 6 in dosage unit form comprising from 0.5 to 1.0 g per dose of alginic acid and sodium alginate as the base for the mucilage.

8.    A mixture comprising the non-aqueous components of a pharmaceutical composition as claimed in any one of claims 1 to 7.

9.    Guaiacol and its pharmaceutically acceptable derivatives for use in the treatment of heartburn or of constipation of the large bowel.

# EUROPEAN SEARCH REPORT

European Patent Office

Application number

EP 80 30 4761

| | DOCUMENTS CONSIDERED TO BE RELEVANT | | CLASSIFICATION OF THE APPLICATION (Int Cl.³) |
|---|---|---|---|
| Category | Citation of document with indication, where appropriate, of relevant passages | Relevant to claim | |
| | FR - A - 2 171 222 (REED & CAPNRICK) <br><br> * page 2, lines 9-16; page 3, lines 11-27; page 3, line 40 - page 4, line 26; page 5, lines 1-10; example 2 * <br><br> & DE - A - 2 305 940 <br><br> -- | 1,2,4, 5,7-9 | A 61 K 9/02 |
| | US - A - 3 422 189 (RIDER) <br><br> * column 1, lines 18-23; column 1, line 49 - column 2, line 16; column 2, lines 57-68 * <br><br> -- | 1,3,6 | TECHNICAL FIELDS SEARCHED (Int. Cl.³) <br><br> A 61 K 9/02 |
| | FR - M - 1031 (DEBARGE) <br><br> * the whole document * <br><br> -- | 1 | |
| A | HAGERS, Handbuch der Pharmazeutischen Praxis,4e edition vol.7, Springer Verlag pages 358-359 <br><br> * pages 2,3,5,8 Alginate: "Guajacolum glycerolicum" * <br><br> ------ | 1-9 | |
| | | | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons <br><br> &: member of the same patent family, corresponding document |

| | | | |
|---|---|---|---|
| X | The present search report has been drawn up for all claims | | |
| Place of search <br> The Hague | Date of completion of the search <br> 09-09-1981 | Examiner <br> CONTET | |

EPO Form 1503.1   06.78